# EUROPEAN PATENT APPLICATION

(11) **EP 2 848 257 A2**
(43) Date of publication of application: **18.03.2015**
(21) Application number: 13788643.8
(22) Date of filing: 07.05.2013
(51) Int. Cl.: A61K 39/395, A61K 38/17, A61K 38/16, A61P 31/04, A61P 31/00

(54) **VACCINE COMPOSITION FOR PREVENTING STAPHYLOCOCCUS AUREUS INFECTION**

(30) Priority: 07.05.2012 US 201261643697 P
(71) Applicant: Mogam Biotechnology Institute, Gyeonggi-do 446-770 (KR); Pusan National University Industry-University Cooperation Foundation, Busan 609-735 (KR)
(72) Inventor: LEE, Bok Luel, Busan 609-751 (KR); KENJI, Kurokawa, Sasebo Nagasaki 859-3215 (JP); JUNG, Dong-Jun, Gimhae-si Gyeongsangnam-do 621-900 (KR); AN, Jang-Hyun, Pohang-si Gyeongsangbuk-do 791-779 (KR); JEON, Yu-Jin, Changwon-si Gyeongsangnam-do 642-050 (KR); KIM, Na Hyang, Busan 607-772 (KR); KAZUE, Takahashi, Cambridge Massachusetts 02139-1229 (US); KIM, Eunmi, Yongin-si Gyeonggi-do 446-799 (KR); AHN, Dong Ho, Yongin-si Gyeonggi-do 446-770 (KR)
(74) Representative: Albutt, Jodie
(86) International application number: PCT/KR2013/003957
(87) International publication number: WO 2013/168965

(57) **Abstract**

The present invention relates to a vaccine composition for preventing *staphylococcus aureus* infection containing, as an active ingredient, a ribitol-phosphate which has been modified only by a β-configuration in *N*-Acetylglucosamine (GlcNAc), a repeating unit of the ribitol-phosphate, or a wall teichoic acid (WTA) containing the repeating unit. The composition according to the present invention contains a coupling motif (an epitope) for an anti-WTA antibody, and thus can be effectively used as a vaccine composition or to prevent *staphylococcus aureus* infection by generating anti-WTA antibody.

## Description

### FIELD OF THE INVENTION

The present invention relates to a vaccine composition for preventing *staphylococcus aureus* infection. More specifically, it relates to a vaccine composition for preventing *staphylococcus aureus* infection comprising, as an active ingredient, a ribitol-phosphate which has been modified only by a β-configuration in *N*-acetylglucosamine (GlcNAc), a repeating unit of the ribitol-phosphate or a wall teichoic acid (WTA) containing the repeating unit.

### BACKGROUND OF THE INVENTION

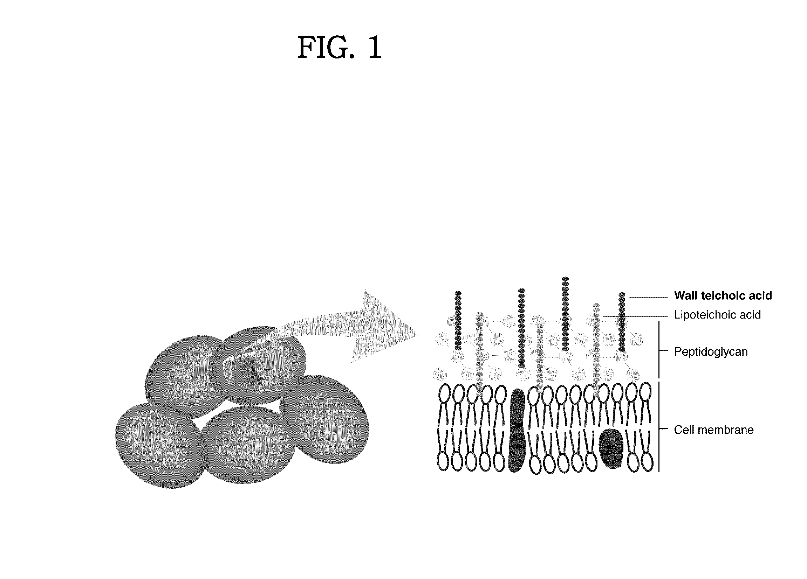

*Staphylococcus aureus* can cause serious infections in the skin, soft tissue and blood stream in the community and in hospitalized patients *(see* Lowy FD, The New England journal of medicine, 339:520-532, 1998). Recent spreading of methicillin-resistant *S*. *aureus* (MRSA) increases the difficulty of treating infections. *S. aureus* is a Gram-positive pathogen with a single cell membrane surrounded by glycopolymers including wall teichoic acid (WTA), peptidoglycan, lipoteichoic acid and capsular polysaccharide (*see* Fig. 1).

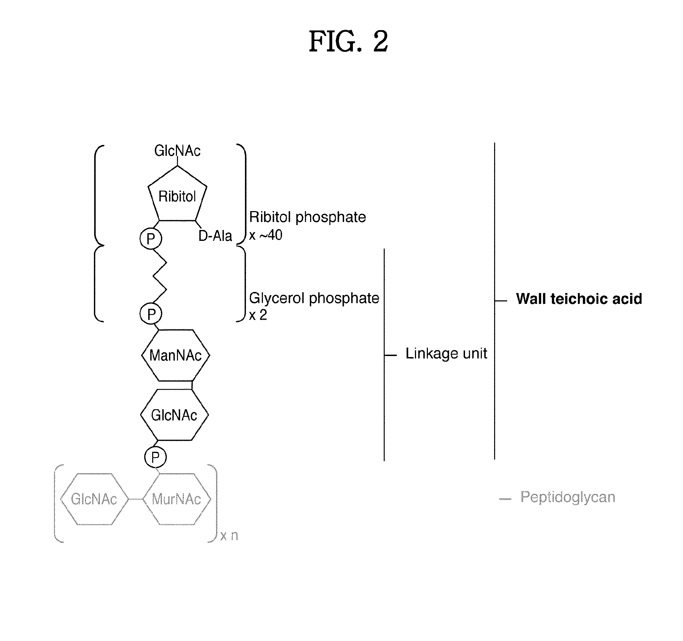

*S. aureus* WTA is a glycopolymer that covalently links to peptidoglycan and is composed of an *N*-acetylmannosamine (ManNAc)-(β-1,3)-*N*-acetylglucosamine (GlcNAc) disaccharide with two glycerol phosphates, followed by 10-40 ribitol-phosphate repeating units *(see* Weidenmaier C et al., Nature reviews, 6:276-287, 2008; Swoboda JG et al., Chembiochem., 11:35-45, 2010). The hydroxyls on the ribitol-phosphate repeating units are modified with cationic D-alanine esters and GlcNAc *(see* Xia G et al., Int. J. Med. Microbiol., 300:148-154, 2010; Fig. 2).

Although *S. aureus* WTA is dispensable for bacterial viability, it is suggested to be involved in the adherence *of S. aureus* to nasal epithelial cells *(see* Weidenmaier C, et al., Nature medicine, 10:243-245, 2004). Also, *S. aureus* WTA is known to induce CD4+ T-cell proliferation in a major histocompatibility complex (MHC) II-dependent manner, which in turn modulates abscess formation in a mouse skin infection model *(see* Weidenmaier C et al., PloS one 5:e13227, 2010).

In the early 1960s, immunochemical studies were conducted by several research groups to determine the cell wall structure of *S. aureus* Copenhagen strain by using rabbit sera. It was discovered that the agglutination activity of rabbit anti-sera was inhibited by *S*. *aureus* WTA or by the purified α-GlcNAc-modified ribitol-phosphate. Based on these observations, the rabbit sera were believed to contain anti-WTA antibodies specific for α-GlcNAc epitopes on WTA (*see* Nathenson SG et al., J. Biol. Chem., 237:3839-3841, 1962; Juergens WG et al., J. Exp. Med., 117:925-935, 1963). In addition, immunization with *S*. *aureus* Copenhagen and NYH-6 strains to human resulted in antibodies recognizing both α-GlcNAc and β-GlcNAc WTAs (*see* Gründling A et al., Proc. Natl. Acad. Sci. USA, 104, 8478-83, 2007).

The exact epitope *of S. aureus* WTA and the specificity of antibodies after *S. aureus* infection are not clearly determined until now and, thus, substantial research efforts have been directed to this topic. However, not much progress has been made due to lack of genetic information regarding GlcNAc transferases involved in the biosynthesis *of S. aureus* WTA and the difficulty of purification of WTA, specifically α-GlcNAc- or β-GlcNAc-WTAs, due to the absence of mutant *S. aureus* lacking α-GlcNAc- or β-GlcNAc-modification of WTA.

Recently, two research groups identified two WTA glycosyltransferases, *TarM* and *TarS,* which are responsible for biogenesis of WAT with α-GlcNAc and β-GlcNAc, respectively (*see* Xia G et al., J. Biol. Chem. 285:13405-13415, 2010; Brown S et al., Proc. Natl. Acad. Sci. U S A 109:18909-18914, 2012). It was also revealed that said two transferases, *TarM* and *TarS*, are capable of carrying out glycosylation of α-GlcNAc and β-GlcNAc residues, respectively, of ribitol-phosphate in WTA, in a UDP-GlcNAc-dependent manner.

Leading to the present invention, the present inventors have endeavored to study on *S*. *aureus* WTA; and have found that β-GlcNAc residue of WTA causes induction of classical complement-dependent opsonophagocytosis *of S. aureus* and the fact that antibodies which recognize WTA β-GlcNAc residues as an epitope are mostly produced in serum.

### SUMMARY OF THE INVENTION

Accordingly, it is therefore an object of the present invention to provide a vaccine composition for preventing *staphylococcus aureus* infection.

It is another object of the present invention to provide a method for preparing anti-WTA antibodies *of S. aureus*.

In accordance with one aspect of the present invention, there is provided a vaccine composition for preventing *staphylococcus aureus* infection comprising a native form of WTA containing a ribitol-phosphate which has been modified by GlcNAc, which comprises, as an active ingredient: (i) a ribitol-phosphate which has been modified only by a β-configuration in GlcNAc and not by α-configuration; (ii) a repeating unit of the ribitol-phosphate which has been modified only by a β-configuration in GlaNAc and not by α-configuration; or (iii) WTA containing the repeating unit of the ribitol-phosphate which has been modified only by a β-configuration in GlaNAc and not by α-configuration.

In accordance with another aspect of the present invention, there is provided a method for preparing anti-WTA antibodies of *S. aureus*, which comprises the steps of: (1) preparing a mutant *S. aureus* containing WTA which has been modified only by a β-configuration in GlaNAc and not by α-configuration by mutating *S. aureus* with a native form of WTA containing GlcNAc-modified ribitol-phosphate; (2) separating and purifying (i) ribitol-phosphate which has been modified only by a β-configuration in GlaNAc and not by α-configuration; (ii) a repeating unit of the ribitol-phosphate which has been modified only by a β-configuration in GlaNAc and not by α-configuration; and (iii) WTA containing the repeating unit of the ribitol-phosphate which has been modified only by a β-configuration in GlaNAc and not by α-configuration from the mutant *S. aureus*; and (3) preparing anti-WTA antibodies by using the ribitol-phosphate, the repeating unit of the ribitol-phosphate or the WTA containing the repeating unit of the ribitol-phosphate as an antigen.

### BRIEF DESCRIPTION OF DRAWINGS

The above and other objects, features and other advantages of the present invention will be more clearly understood from the following detailed description taken in conjunction with the accompanying drawings, in which:
Fig. 1 is a schematic drawing illustrating a cell wall structure *of S. aureus*.
Fig. 2 is a schematic drawing illustrating a wall teichoic acid (WTA) *of S. aureus*.
Fig. 3 is a schematic drawing illustrating *S. aureus* WTA, a residue which has been modified in the WTA and a related gene. In the drawing, GlcNAc is an abbreviation for *N*-acetylglucosamine, ManNAc is an abbreviation for *N*-acetylmannoseamine, D-Ala is an abbreviation for D-alanine and Pi is an abbreviation for phosphate.
Fig. 4 is a result of flow cytometric analysis comparing anti-WTA IgG binding, C3 deposition and C4 deposition on parental *S. aureus* strains and mutants (Δ*TarS*, Δ*TarM*, Δ*dltA*, or Δ*tagO*).

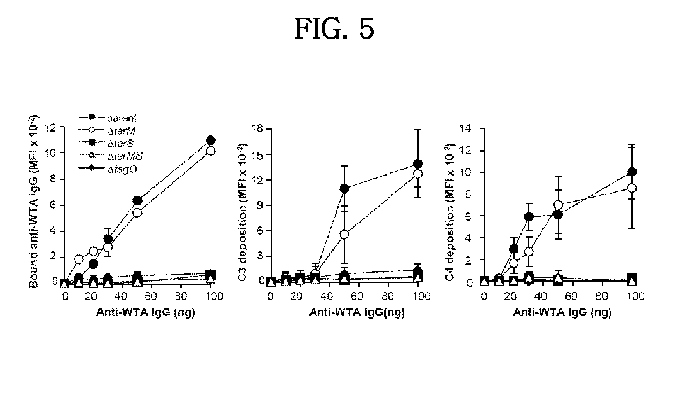

Fig. 5 is an analysis result showing anti-WTA IgG binding, C3 deposition and C4 deposition with respect to anti-WTA IgG concentration.
Fig. 6 shows complement-mediated-opsonophagocytosis induced by the parental strains and mutants of the present invention, which shows the number of engulfed *S*. *aureus*/100 human polymorphonuclear neutrophils (PMNs)
Fig. 7 is a WTA PAGE analysis result of WTAs separated from the parental strains and mutants.
Fig. 8 is an ELISA result showing the binding ability of each WTA of the parental strains and mutants of the present invention to anti-WTA IgG.

### DETAILED DESCRIPTION OF THE INVENTION

As used herein, the term "wall teichoic acid (WTA)" refers to one of the components found in the cell wall of *Staphylococcus aureus* which is a glycopolymer of ribitol phosphate having an *N*-acetylmannosamine(ManNAc)-(β-1,3)-*N*-acetylglucosamine(GlcNAc) disaccharide linker with two glycerol phosphates followed by 10-40 ribitol-phosphate repeating unit. In a native form of *S. aureus* WTA, ribitol-phosphate is substituted with GlcNAc and D-alanine, and said GlcNAc is substituted in an α-configuration and/or β-configuration.

Also, as used herein, the term "α-GlcNAc" or "α-GlcNAcylation" refers to a condition in which GlcNAc is bonded to the ribitol hydroxy group of WTA in an α-configuration. Similarly, "β-GlcNAc" or "β-GlcNAcylation" refers to a condition in which GlcNAc is bound to ribitol hydroxyl group of WTA in a β-configuration. Moreover, "α-GlcNAc deficient" or "α-GlcNAcylation deficient" refers to a condition in which GlcNAc is not bound to ribitol-phosphate in an α-configuration. On the other hand, "β-GlcNAc deficient" or "β-GlcNAcylation deficient" refers to a condition in which GlcNAc is not bound to ribitol-phosphate in a β-configuration.

Further, as used herein, the term "anti-WTA antibody" refers to an antibody specific for WTA. Said antibody protects its host against staphylococcal infection by inducing complement-mediated-opsonophagocytosis.

The present invention begins with confirmation of WTA binding motif (epitope) which can be recognized by anti-WTA antibodies. Based on the experimental results using α-GlcNAc WTA-deficient Δ*TarM* mutants, β-GlcNAc WTA-deficient Δ*TarS* mutants and double mutants Δ*TarMS* which have neither α-GlcNAc nor β-GlcNAc residues, the present invention suggests that GlcNAc residues bound to ribitol-phosphate of WTA in β-configuration are epitopes which dictate the anti-WTA IgG-mediated complement activation that leads to opsonophagocytosis.

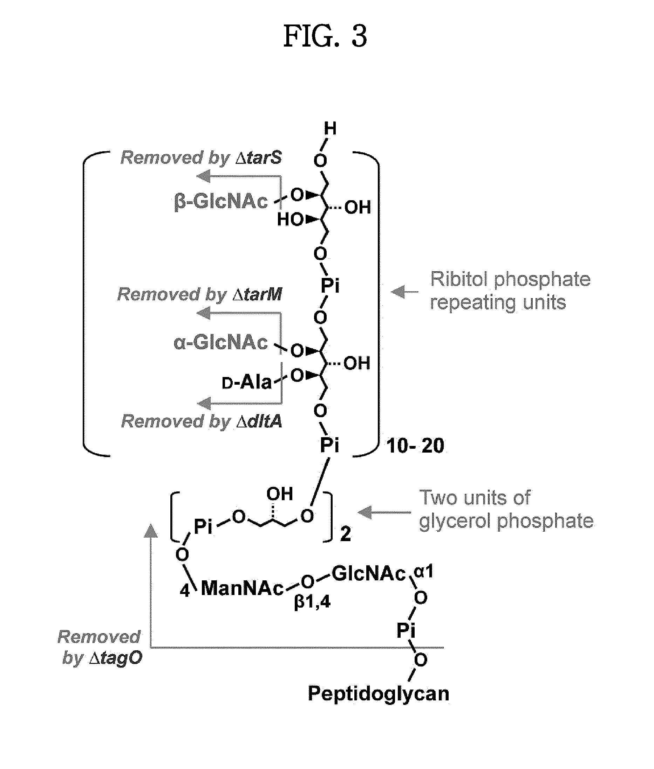

Specifically, the present invention shows that anti-WTA antibodies bind on the β-GlcNAc WTAs separated from the parental strains, *ΔTarM* mutant strains and *ΔdltA* mutant strains, but not on the WTAs separated from *ΔTarMS* double mutant strains, *ΔTarS* mutant strains and *ΔtagO* mutant strains which are deficient in WTA β-GlcNAc residue *(see* Example 2). Also, C3 and C4 deposition was observed in β-GlcNAc WTAs such as the parental strains, *ΔTarM* mutant strains and *ΔdltA* mutant strains, but not in the WTAs separated from *ΔTarMS* double mutant strains, *ΔTarS* mutant strains and *ΔtagO* mutant strains which are deficient in WTA β-GlcNAc residue. These results indicate that the β-GlcNAc WTAs induce complement activation *(see* Fig. 2). Further, the present invention shows that the *ΔTarM* and *ΔdltA* mutant strains were engulfed by human polymorphonuclear neutrophils (PMNs), whereas *ΔTarMS* mutant strains, *ΔTarS* mutant cells and *ΔtagO* mutant strains were not engulfed by PMNs. These results indicate that β-GlcNAc modification of WTA is essential for the opsonophagocytosis *(see* Fig. 3).

Thus, the present invention provides a proof that anti-WTA antibodies bind to β-GlcNAc residues, among native WTAs substituted with α- or β-GlcNAc, and improves efficiency of host immune responses.

Accordingly, the present invention provides a vaccine composition for preventing *staphylococcus aureus* infection comprising a native form of WTA containing a ribitol-phosphate which has been modified by GlcNAc, which comprises, as an active ingredient: (i) a ribitol-phosphate which has been modified only by a β-configuration in GlcNAc and not by α-configuration; (ii) a repeating unit of the ribitol-phosphate which has been modified only by a β-configuration in GlaNAc and not by α-configuration; or (iii) WTA containing the repeating unit of the ribitol-phosphate which has been modified only by a β-configuration in GlaNAc and not by α-configuration.

The WTA containing a ribitol-phosphate which has been modified by GlcNAc, which comprises, as an active ingredient: (i) a ribitol-phosphate which has been modified only by a β-configuration in GlcNAc and not by α-configuration; (ii) a repeating unit of the ribitol-phosphate which has been modified only by a β-configuration in GlaNAc and not by α-configuration; or (iii) WTA containing the repeating unit of the ribitol-phosphate which has been modified only by a β-configuration in GlaNAc and not by α-configuration employed in the present invention may be obtained by inhibiting *TarM* which is responsible for α-GlcNAcylation of ribitol-phosphate in *S. aureus* WTA, thereby preventing the ribitol-phosphate of WTA from being modified by α-configuration in GlcNAc. In one particular example of the present invention, α-GlcNAc WTA-deficient mutant strains can be obtained by deletion of *TarM* gene, which encodes glycosyltransferase *TarM,* from M0107 strains that is deficient for IgG-binding protein A *(see* Oku Y et al., Journal of bacteriology, 191:141-151,2009).

A vaccine composition according to the present invention may be used for the prevention of an infection caused by *staphylococcus aureus*, more specifically, an infection caused by a kidney dialysis catheter or an infection caused by a surgical operation, a skin and soft tissue infection (SSTI), pneumonia, septicemia or acute respiratory tract infection.

The *staphylococcus aureus* may be methicillin-resistant *staphylococcus aureus* (MRSA) or pathogenic *staphylococcus aureus*, but not limited thereto.

In addition to the WTA, the vaccine composition of the present invention may further comprise a pharmaceutically acceptable carrier, a diluent and/or an adjuvant.

The type of carriers employable in the present invention will depend on factors including the mode and the route of administration and its standard drug composition. Examples of the carrier may include a carrier protein (e.g., bovine serum albumin (BSA), ovalbumin (OVA), human serum albumin (HSA) and keyhole limpet hemocyanin (KLH)), a solubilizing agent (*e.g*., ethanol, Polysorbates and Cremophor EL™), an agent for making isotonicity, a preservative, an antioxidizing agent, an excipient (*e.g*., lactose, starch, crystalline cellulose, mannitol, maltose, calcium hydrogen phosphate, light silicic acid anhydride and calcium carbonate), a binder (*e.g*., starch, polyvinylpyrrolidone, hydroxypropyl cellulose, ethyl cellulose, carboxyl methyl cellulose, and gum Arabic), a lubricant *(e.g.,* magnesium stearate, talc, and hardened oils) and a stabilizer *(e.g.,* lactose, mannitol, maltose, Polysorbates, macrogols, and polyoxyethylene hardened castor oils). If necessary, glycerin, dimethylacetamide, 70% sodium lactate, a surfactant or a basic substance (*e.g*., sodium hydroxide ethylenediamine, ethanolamine, sodium bicarbonate, arginine, meglumine and trisaminomethane) may be added. Specifically, the vaccine composition of the present invention may be added with a KLH solution (Calbiotec, 125 mg/l mL 50% glycerol solution) known in the art, as a carrier protein, in order to improve its antigenicity.

The type of diluents employable in the present invention will depend on factors including the mode and the route of administration and its standard drug composition. Examples of the diluent include water, normal saline, phosphate buffered normal saline and sodium bicarbonate solution.

The type of adjuvants employable in the present invention will depend on factors including the mode and the route of administration and its standard drug composition. Examples of the adjuvant include cholera toxin, *E. coli* heat labile enterotoxin (LT), liposome and an immune stimulating complex (ISCOM).

It is understood that the administration route should be determined in light of various relevant factors including the condition, age, body weight and sex of the subject to be treated, and may be administered orally or parenterally (*e.g*., intravenously, intraarterially and topically), preferably in the form of parenteral administration.

Formulations for oral and parental administration and manufacturing method thereof are well known in the art. Formulations for oral and parental administration may be formulated in accordance with conventional methods known in the art, for example, by mixing with the aforementioned pharmaceutically acceptable carrier. Examples of oral formulations include solvents, tablets, granules, powders and solid or liquid formulations such as capsules. Examples of parenteral formulation include solvents, suspensions, ointments, suppositories, eye drops, ear drops, nasal sprays. The composition of the present invention may be formulated into a sustained release form by using biodegradable polymers *(e.g.,* poly-D,L-lactide-co-glycolide or polyglycolide) as a bulk matrix *(see* U.S. Pat. Nos. 5,417,986, 4,675,381, and 4,450,150). If the composition is to be administered orally, flavorings and/or colors may be added. Suitable pharmaceutical excipients and diluents, as well as pharmaceutical necessities for their use, are described, *e.g*., in Remington's Pharmaceutical Sciences.

Dosages of the vaccine composition administered in practicing the present invention will depend on factors including the type of adjuvant co-administered, the mode and frequency of administration and the desired effect. In general, the composition can be administered to an adult in amounts ranging from 1 µg to 100 mg of WTA per dose. If adjuvants are co-administered with the vaccine composition, the composition can be administered to an adult in amounts ranging from 1 µg to 1 mg of WTA per dose. The composition may be administered in divided doses, if necessary. For example, vaccination may be given in 4 doses, *i.e*., an initial dose and three booster doses with an interval of one week. Optionally, the first and the second booster doses may be same formulation and respectively given 8 to 12 weeks and 16 to 20 weeks after the first immunization.

Further, the present invention provides a method for preparing anti-WTA antibodies of *S. aureus*, which comprises the steps of: (1) preparing a mutant *S. aureus* containing WTA, which has been modified only by a β-configuration in GlaNAc and not by α-configuration, by mutating *S. aureus* with a native form of WTA containing GlcNAc-modified ribitol-phosphate; (2) separating and purifying (i) ribitol-phosphate which has been modified only by a β-configuration in GlaNAc and not by α-configuration; (ii) a repeating unit of the ribitol-phosphate which has been modified only by a β-configuration in GlaNAc and not by α-configuration; and (iii) WTA containing the repeating unit of the ribitol-phosphate which has been modified only by a β-configuration in GlaNAc and not by α-configuration from the mutant *S. aureus*; and (3) preparing anti-WTA antibodies by using the ribitol-phosphate, the repeating unit of the ribitol-phosphate or the WTA containing the repeating unit of the ribitol-phosphate as an antigen.

In Step (1), a mutant *S. aureus* containing WTA which has been modified only by a β-configuration in GlaNAc and not by α-configuration is prepared by mutating *S. aureus* with a native form of WTA containing GlcNAc-modified ribitol-phosphate. As described above, Step (1) may be accomplished by inhibiting glycosyltransferase *TarM* to prevent α-GlcNAc-modification of ribitol-phosphate in WTA. In one particular example, α-GlcNAc WTA-deficient mutant strains can be obtained by deletion of *TarM* gene, which encodes glycosyltransferase *TarM,* from M0107 strains that is deficient for IgG-binding protein A *(see* Oku Y et al., Journal of bacteriology, 191:141-151, 2009).

Also, in Step (2) of the present invention, separation and purification of (i) ribitol-phosphate which has been modified only by a β-configuration in GlaNAc and not by α-configuration; (ii) a repeating unit of the ribitol-phosphate which has been modified only by a β-configuration in GlaNAc and not by α-configuration; and (iii) WTA containing the repeating unit of the ribitol-phosphate which has been modified only by a β-configuration in GlaNAc and not by α-configuration from the mutant *S. aureus* takes place. This process may be carried out according to any conventional methods well known in the art, for example, the method disclosed in Shiratsuchi A et al., Immunology, 129:268-277, 2010. Further, once WTA is obtained, the ribitol-phosphate repeating units may be separated and purified there from, and also the ribitol-phosphate may be further separated and purified from the ribitol-phosphate repeating units.

Also, in Step (3) of the present invention, anti-WTA antibodies are prepared by using the separated and purified ribitol-phosphate, the repeating unit of the ribitol-phosphate or the WTA containing the repeating unit of the ribitol-phosphate as an antigen. This process may be accomplished by using any conventional methods of preparing antibodies from an antigen well known in the art, for example, immunization by inoculating the separated and purified ribitol-phosphate, the repeating unit of ribitol-phosphate or the WTA containing a repeating unit of ribitol-phosphate to a mammal.

The anti-WTA antibodies thus prepared may be used for the prevention of an infection caused by *Staphylococcus aureus*, more specifically, an infection caused by a kidney dialysis catheter or an infection caused by a surgical operation, a skin and soft tissue infection (SSTI), pneumonia, septicemia or acute respiratory tract infection.

The *S. aureus* may be methicillin-resistant *S. aureus* (MRSA) or pathogenic *S. aureus*, but not limited thereto.

Additionally, the present invention provides a method for preventing *staphylococcus aureus* infection, which comprises administering to a mammal the separated and purified ribitol-phosphate, the repeating unit of the ribitol-phosphate or the WTA containing the repeating unit of the ribitol-phosphate obtained in Step (2) or the anti-WTA antibodies obtained in Step (3).

Further, the present invention provides a method for preparing a vaccine composition for preventing *staphylococcus aureus* infection using the separated and purified ribitol-phosphate, the repeating unit of the ribitol-phosphate or the WTA containing the repeating unit of the ribitol-phosphate obtained in Step (2) or the anti-WTA antibodies obtained in Step (3).

Hereinafter, the present invention is described more specifically by the following examples, but these are provided only for illustration purposes and the present invention is not limited thereto.

### Example 1: Preparation of Mutant S. aureus

*S. aureus* WTA contains a long chain of ManNAc and GlcNAc disaccharide linker with two glycerol phosphates followed by ribitol phosphate repeating units which are substituted with α- or β-GlcNAc and D-alanine, and is linked to peptidoglycan *(see* Fig. 3).

In this example, α-GlcNAc WTA deficient, β-GlcNAc WTA deficient and D-alanine deficient *S. aureus* mutants were prepared so as to determine the epitope of anti-WTA antibodies.

### <1-1> Preparation of IgG-binding Protein Deficient Mutant Strain (M0107)

Prior to determining the epitope of anti-WTA antibodies, a *spa* gene which is responsible for IgG-binding protein A was deleted from RN4220 strain *(see* Novick RP et al., Embo. J., 12:3967-3975. 1993) to prepare IgG-binding protein A deficient mutant strains (M0107) (*see* Oku Y, et al., Journal of bacteriology, 191:141-151, 2009) so as to prevent the anti-WTA antibodies from binding with IgG-binding protein A.

Specifically, a *spa* gene of RN4220 strain was replaced by a phleo resistance coding sequence via double crossover recombination using the following primers:
spa-P1: GGGTCTAGAAAAAAGTCAAGCCTGAAGTCG (SEQ ID No: 1);
spa-P2: TATTGGATCCAAAGTGGGGCTTTGAATGTG (SEQ ID No: 2);
spa-P3: CCCGGGTACCTGCAGCGTTATTAGCTGGAC (SEQ ID No: 3);
spa-P4: GGGGAATTCTAATTGGTGCAACTGGGACA (SEQ ID No: 4);
Phleo-P1: GGATCCAATAGACCAGTTGCA (SEQ ID No: 5); and
Phleo-P3: GGTACCCGGGCGATTGCTGAA (SEQ ID No: 6).

### <1-2> Preparation of β-GlcNAc Deficient Mutant Strain (T803)

As shown in Fig. 3, a *TarS* gene was deleted from the M0107 strain prepared in Example <1-1> to prepare β-GlcNAc deficient mutant strains (T803).

Specifically, pSF151 plasmid (km^{r}) having an open reading frame region of *TarS* gene (*see* Tao L et al., Gene, 120:105-110, 1992) was inserted in the *Bam*HI and *Eco*RI site of *TarS* gene which is responsible for coding of β-GlcNAc transferase, to disrupt the *TarS* gene. Subsequently, the disrupted *TarS* gene was amplified by PCR using the following primers and then confirmed by electrophoretic analysis:
SA0248-F-*Bam*HI: CAACTGGATCCAAATTCTGGTGGTCCAGGT (SEQ ID No: 7); and
SA0248-R-*Eco*RI: TTTCGAATTCGCGTAGTGCAACAATGGTCGT (SEQ ID No: 8).

### <1-3> Preparation of α-GlcNAc Deficient Mutant Strain (T790)

As shown in Fig. 3, a *TarM* gene was deleted from the M0107 strain prepared in Example <1-1> to prepare α-GlcNAc deficient mutant strains (T790).

Specifically, pMutinT3 (erm^{r}) having an open reading frame region of *TarM* gene *(see* Vagner V et al., Microbiology (Reading, England) 144 (Pt 11):3097-3104, 1998) was inserted in the *Hind*III and *Bam*HI site of *TarM* gene which is responsible for coding of α-GlcNAc transferase, to disrupt the *TarM* gene. Subsequently, the disrupted *TarM* gene was amplified by PCR using the following primers and then confirmed by electrophoretic analysis:
*TarM*-F1-*Hind*III: ATGATGCGGACATACCTGCT (SEQ ID No: 9); and
*TarM*-R2-*Bam*HI: CACGGATCCTAAATGCACCCGTATCATCGAA (SEQ ID No: 10)

### <1-4> Preparation of α-GlcNAc and β-GlcNAc Deficient Mutant Strain (T807)

As shown in Fig. 3, both *TarM* and *TarS* genes were deleted from the M0107 strain prepared in Example <1-1> to prepare α- and β-GlcNAc deficient mutant strains (T807). The procedures of Examples <1-2> and <1-3> were repeated to prepare the mutant strains.

### <1-5> Preparation of D-alanine Deficient Mutant Strain (T861)

As shown in Fig. 3, a *dltA* gene was deleted from the strain M0107 prepared in Example <1-1> to prepare D-alanine deficient mutant strains (T861).

Specifically, the internal region of a dltA gene (positions 46 to 694; position 1 is the translation initiation site) was amplified by PCR and was inserted into pMutinT3 vector, resulting in plasmid pT0793. The plasmid pT0793 was transfected with pT0793, and then strains resistant to erythromycin were identified to obtain mutant strains with disrupted *dltA* gene (T861). Disruption of the *dltA* gene and insertion of the targeting plasmid into the desired chromosomal locus were confirmed by Southern blot analysis.

### <1-6> Preparation of WTA Deficient Mutant Strain (T258)

As shown in Fig. 3, a *tagO* gene was deleted from the strain M0107 prepared in Example <1-1> to prepare WTA deficient mutant strains (T861).

Specifically, the internal region of a *tagO* (positions 36 to 645) was amplified by PCR and was inserted into pMutinT3 vector, resulting in plasmid pT0702. The plasmid pT0793 was transfected with M0107 strain to obtain mutant strains with disrupted *tagO* gene (T861).

Information of said mutant strains is summarized in Table 1 below.

**[Table 1]**

| Strain | Example | Genotype | Phenotype |
|---|---|---|---|
| RN4220 | | NCTC8324-5, restriction mutant | |
| M0107 | <1-1> | RN4220 *Δspa::phleo* | IgG-binding protein A-disrupted |
| T803 | <1-2> | M0107Δ*TarS::km* | WTA-β-GlcNAc-disrupted |
| T790 | <1-3> | M0107ΔT*arM*::*erm* | WTA-α-GlcNAc-disrupted |
| T807 | <1-4> | M0107Δ*TarM*::*erm*Δ*TarS*::*km* | WTA-α,P-GlcNAc-disrupted |
| T861 | <1-5> | M0107Δ*dltA*::*erm* | WTA D-alanine-disrupted |
| T258 | <1-6> | M0107 Δ*tagO*::*erm* | WTA-disrupted |

Among said strains, the parental strains and *ΔdltA* mutant strains contain both α- and β-GlcNAc modifications on WTAs. But, since *ΔTarS* mutant strains cannot synthesize β-modified WTA and *ΔTarM* mutant strains cannot synthesize α-GlcNAc modified WTA, *ΔTarS* and *ΔTarM* have either α-GlcNAc or β-GlcNAc modification on WTAs, respectively. However, *ΔTarM* and *ΔTarS* double mutant strains (*ΔTarMS*) and *ΔtagO* mutant strains have no GlcNAc substitutions on their WTAs.

### Example 2: Binding of Mutant Strains and Anti-WTA Antibodies

In order to confirm the binding site of WTA to which anti-WTA antibodies bind, anti-WTA antibodies were prepared and mutant strains obtained in Example 1 were assayed for their binding site for the anti-WTA antibodies.

### <2-1> Separation and Purification of WTA

In order to separate WTA, a mutant strain (T384) which is peptidoglycan O-acetyltransferase (*oatA*) gene and lipoprotein diacylglyceryl transferase (*lgt*) deficient was prepared from *S. aureus* RN4220 strains. Specifically, each of T363 strain (RN4220 Δ*lgt*::*phleo*) and T002 strain (RN4220 Δ*oatA*::*erm*) was obtained from RN4220 strains by using the methods disclosed in Nakayama M et al., J. Immunol., 189 (12), 5903-5911, 2012; and Park KH et al., J. Biol. Chem., 285 (35), 27167-2775, 2010, and strains were transduced using phage 80 alpha. Since *lgt* mutants are removed by lipoprotein of *S. aureus*, lipoprotein free WTA can be obtained from these strains. Also, *oatA* mutants sensitize *S*. *aureus* peptidoglycan to lysozyme.

The T384 strain obtained above was incubated in LB medium, centrifuged at 6,000 rpm for 15 minutes to remove the supernatant. Then, pellet was suspended in 10 mL of sodium citrate (20 mM, pH 4.5), centrifuged at 15,000 rpm for 5 minutes to remove the supernatant. The pellet was suspended in 20 mL of sodium citrate supplemented with 1M NaCl (20 mM, pH 4.5), the cells were broken with glass beads (12g/L) using a beads blender. The broken cells were then moved to a 50 mL tube, centrifuged at 3,000 rpm for 10 minutes to remove the supernatant, and the pellet was collected.

The pellet thus obtained was suspended in 20 mL of 20 mM sodium citrate (0.5 SDS, pH 4.5), heated at 60°C for 30 minutes and centrifuged at 15,000 rpm for 10 minutes to remove the supernatant. The above procedure was repeated 5 times to remove SDS completely, and the pellet was washed with 20 mM Tris (pH 7.0) to make the pH neutral. Subsequently, the pellet was suspended in 5 mL of 20 mM Tris (pH 7.0), added with 50 µL of 1 M CaCl₂ and 50 µL of trypsin (20 mg/mL) and incubated at 37°C for 12 hours. The culture solution thus obtained was then centrifuged at 15,000 rpm for 10 minutes to remove the supernatant, and the pellet was suspended in 20 mL of distilled water for injection to wash the pellet. After the washing process was repeated 5-6 times, the pellet was suspended in 1 mL of distilled water for injection, and subjected to lyophilization.

The lyophilisate containing WTA was suspended in Tris (pH 7.0, 1 mL/50 mg lyophilisate), treated with lysostaphin (Sigma Aldrich; 0.1 mg/10 mg lyophilisate), and allowed to react at 37°C for 12 hours. Hen egg-white lysozyme (Bioshop, 0.1 mg/ 10 mg lyophilisate) was added to the lyophilisate, followed by a further reaction for 12 hours. Upon completion of reaction, the compound was heated at 95°C for 10 minutes to deactivate enzymes and centrifuged at 15,000 rpm to collect the supernatant. The supernatant thus obtained was filtered using 0.45 µm membrane filter. Then, monomeric peptidoglycan-linked WTA was purified by carrying out an ion exchange chromatography under the following conditions.
- Column: Hitrap Q FF™ 5mL
- Flow rate: 0.5 mL/min
- Loading sample: Filtered fraction 2 mL
- Buffer: Buffer A - 20 mM Tris-HCl (pH 7.0)
   Buffer B - 20 mM Tris-HCl + 1M NaCl (pH 7.0)
- Absorbance: λ 220 nm
- Sensitivity: 0.5 AUFs
- Gradient: 50-min 0 ∼ 100%
- Fraction: 1 mL/tube

The fraction obtained from the chromatography was mixed with cold acetone (fraction:acetone = 1:3), precipitated and then centrifuged at 15,000 rpm for 20 minutes to remove the supernatant. Subsequently, the precipitate was hardened by spraying 40 to 50 µL of acetone thereto, collected in a 1.5 mL tube and dried for 1 hour to remove acetone. The dried WTAwas dissolved in 500 µL of distilled water for injection, and then subjected to lyophilization.

### <2-2> Separation and Purification of Anti-WTA Antibodies

Anti-WTA antibodies were separated from commercially available human intravenous immunoglobulin (IVIG, Green Cross Corporation, total 400 mg) and purified by using the WTA obtained in Example <2-1>.

Specifically, WTA was spotted onto a nitrocellulose membrane (10 x 90 mm, Whatman, pore 0.45 µm), and baked at 100°C for 1 hour. The membrane was washed with buffer A (20 mM Tris-HCl, pH 7.4, 150 mM NaCl), and blocked with buffer D (20 mM Tris-HCl, pH 7.4, 150 mM NaCl and 1% BSA) at 4°C for 2 hours. The nitrocellulose membrane was incubated with 50 mg of IVIG in 40 mL of buffer E (10 mM Tris, pH 7.4, 140 mM NaCl) at 4°C for 2. After washing with buffer A, bound antibodies were eluted with 1 mL of 0.1 M glycine (pH 2.8) and immediately neutralized with 1 M KOH to pH 7.5. Glycine in the eluted IgG fraction was removed by 3-times repeated operation of Vivaspin 20 (Sartorius) with buffer A.

Subsequently, to remove the anti-peptidoglycan IgGs, the obtained IgG fraction was incubated at 4°C for 2 hours with *S. aureus* Δ*spa* and Δ*tagO* double mutant strains that were pre-fixed with formaldehyde. The *S. aureus* double mutant strains were pelleted with centrifugation, and the supernatant was collected, concentrated by Vivaspin 20, and used as a purified anti-WTA IgG fraction.

### <2-3> Analysis of Binding between Mutant Strains and Anti-WTA IgG

Binding between mutant strains prepared in Example 1 and anti-WTA IgG was analyzed using flow cytometry analysis.

Specifically, ethanol fixed mutant strains of Examples <1-1> to <1-6> (4 µL of a suspension; OD₆₀₀= 3) were incubated with human serum in 20 µL of an incubation buffer [10 mM Tris-HCl (pH 7.4), 140 mM NaCl, 10 mM CaCl₂ and 1% BSA] at 4°C for 2 hours. The incubated strains were washed, and incubated with mouse anti-human IgG mAb (Sigma, diluted 1:200) as the primary antibody, followed by goat F(ab')₂ anti-mouse IgG antibodies conjugated with fluorescein 5-isothiocyanate (FITC) (Beckman coulter, diluted 1:200) as the secondary antibody. The incubated strains were appropriately washed with washing buffer (10 mM Tris-HCl (pH 7.4), 140 mM NaCl, 10 mM CaCl₂ and 0.05% Tween 20). Then, the washed strains were sonicated for 15 seconds to disperse clumped cells before flow cytometry analyses (Accuri C6, Beckman Coulter).

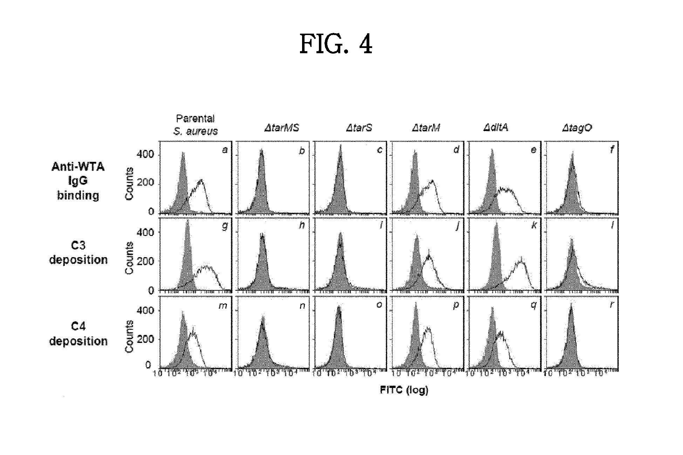

The result of flow cytometry are shown in Figs. 4a to 4f. The gray areas of Figs. 4a to 4f are the results for ethanol-killed *S. aureus* mutant strains incubated without anti-WTA IgG, and the black lines indicate the results for ethanol-killed *S. aureus* mutant strains incubated with anti-WTA IgG.

As shown in Fig. 4, the purified anti-WTA-IgG recognized the parental strains M0108(a), *ΔTarM* mutant strains (d) and *ΔdltA* mutant strains (e), but failed to recognize *ΔTarMS* double mutant strains (b), *ΔTarS* (c) and *ΔtagO* mutant strains (f). These results indicate that β-GlcNAc residues *of S. aureus* is the binding motif which allows them to bind with anti-WTA antibodies.

### <2-4> C3 and C4 Deposition Essay

According to the classical complement pathway, antibody-antigen complexes induce complement activation which cause complement C3 and C4 deposition. Thus, the anti-WTA IgG binding to the *S. aureus* cells and complement activation can be determined by investigating C3 and C4 deposition. In this example, anti-WTA IgG-mediated complement C3 and C4 deposition on mutants obtained in Examples <1-1> to <1-6> was observed using flow cytometric analysis.

Specifically, adult serum obtained from healthy adults was treated with M0107 strains prepared in Example <1-1> according to the method disclosed in Jung DJ et al., J. Immunol., 189:4951-4959, 2012 to prepare *S. aureus*-treated adult serum. Meanwhile, the mutant strains prepared in Examples <1-1> to <1-6> were incubated at 37°C for 12 hours until their OD₆₀₀ value reached 1 to 1.2, washed with 1 mL of PBS 2 times and then fixed acetone. The fixed strains were washed with incubation buffer (10 mM Tris, 140 mM NaCl (pH 7.4), 10 mM CaCl₂ and 1% BSA) 2 times. Subsequently, each of the human sera and mutant strains was allowed to react with anti-WTA antibodies at 37°C for 1 hour and washed with 100 µL washing buffer (10 mM Tris, 140 mM NaCl (pH 7.4) and 10 mM CaCl₂) 2 times. For C3 deposition, as the primary antibody, 20 µL of mouse anti-human C3 IgG conjugated with fluorescein isothiocyanate (FITC) was subjected to a reaction at 4°C for 1 hour. For C4 deposition, 20 µL of mouse anti-human C4 IgG (diluted 1:500) was added thereto as the primary antibody, subjected to a reaction at 4°C for 1 hour, added with 20 µL anti-mouse IgG conjugated with FITC (diluted 1:200) as the secondary antibody and subjected to a reaction at 4°C for 1 hour. After the reaction with antibodies, mutant strains were washed with 100 µL washing buffer 2 times, and C3 and C4 depositions was tested using a flow cytometer.

The results are shown in Fig. 4g to r. The gray area in Fig. 4g to r indicate C3 and C4 depositions in *S. aureus* without anti-WTA IgG, and the black line in Fig 4g to r indicate C3 and C4 depositions in *S. aureus* with anti-WTA IgG.

As shown in Fig. 4g to r, C3 and C4 deposition was observed on *S. aureus* cells which can synthesize WTA with β-GlcNAc configuration, such as the parental strains (g, m), *ΔTarM* (j, p) and *ΔdltA* (k, q) mutant cells. These results indicate that the β-GlcNAc residue of ribitol-phosphate in WTA is required for anti-WTA IgG-mediated complement activation.

### <2-5> Anti-WTA Antibody Binding and C3 and C4 Deposition Depending on Anti-WTA IgG Concentration

The procedure of Example <2-4> was repeated to analyze anti-WTA antibody binding and C3 and C4 depositions, except that the concentration of anti-WTA antibody was changed.

The dose response of anti-WTA IgG showed that anti-WTA antibody binding and C3 and C4 deposition on the parental strains and *ΔTarM*, which have β-GlcNAc residues, were increased in a dose-dependent manner. However, anti-WTA antibody binding and C3 and C4 deposition of *ΔTarS*, *ΔTarMS* and *ΔtagO* mutant strains, which are deficient in β-GlcNAc residue remained unchanged. These results indicate that the β-GlcNAc residue of ribitol-phosphate in WTA is required for anti-WTA antibody binding complement activation.

### Example 3: Complement-mediated-opsonophagocytosis Induced by Mutant Strains

Because anti-WTA IgG specifically induced C3 deposition on β-GlcNAc WTA-synthesizing *S. aureus* cells, it was assumed that C3 opsonized strains can easily be engulfed by the human polymorphonuclear leukocytes (PMNs). To quantify the *S. aureus* cells engulfed by the PMNs, the number of FITC-labeled bacteria engulfed by 100 PMNs was counted under a fluorescent microscope.

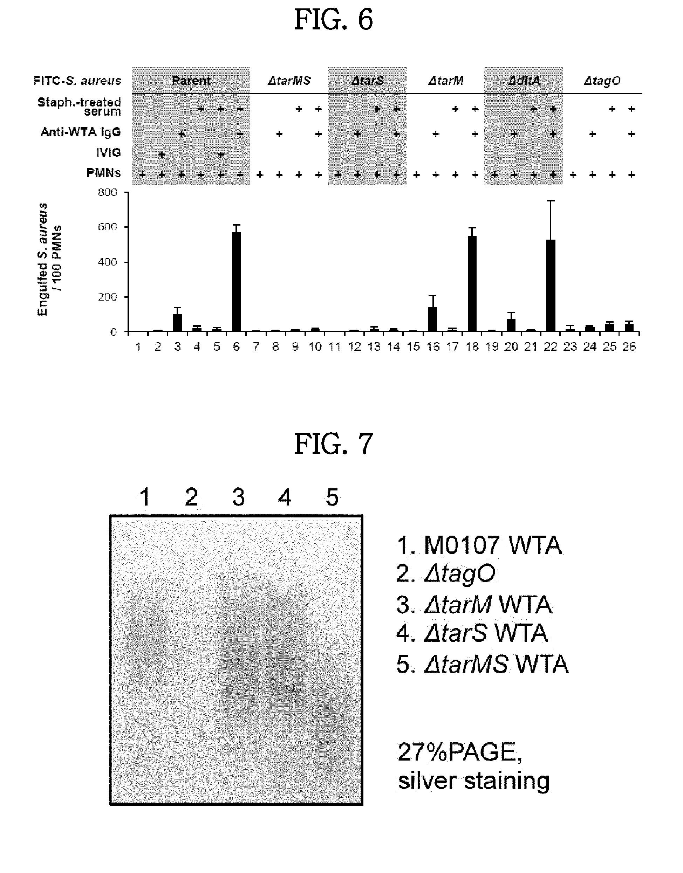

Specifically, M0107 (parental strain) obtained in Example <1-1> and mutant strains obtained in Examples <1-2> to <1-6> which were killed with ethanol, were killed with 70% ethanol, labeled with 0.1 mM FITC (Sigma) in 0.1 M Na₂CO₃ buffer (pH 8.5) at room temperature for 30 minutes, and resuspended in Hank's balanced salt solution (HBSS). Subsequently, the FITC-labeled bacteria (equivalent to 1.5 x 10⁷ CFU) were opsonized with 10% *S. aureus*-treated sera. The opsonized cells were added with anti-WTA antibodies (50 ng). Meanwhile, the peripheral blood mononuclear cells were isolated from healthy donors using Polymorphprep solution (Nycomed Pharm As, Torshov, Norway) according to the method disclosed in Jung DJ et al., J. Immunol. 189:4951-4959, 2012, and the PMNs were obtained via a trypan blue dye exclusion test. Then, the PMN suspension (1.5 x 10⁵ cells, 35 µL) was added to 5 µL of the opsonized bacteria (3.7 x 10⁶ CFU: multiplicity of infection ∼ 25) and incubated at 37°C for 60 minutes. The phagocytosed bacteria cells per 100 PMNs were counted under fluorescent phase-contrast microscopy. The results are shown in Fig. 6, and data are represented as the means ± SD of the results of three independent experiments (p < 0.05).

As shown in Fig. 6, in the absence of *S. aureus*-treated serum, the anti-WTA IgG alone increased PMN-mediated phagocytosis from 1 ± 1 (column 1) to 96 ± 44 (column 3), indicating that the phagocytosis was induced in PMNs via the IgG-Fc receptors (FcγRs). In identical conditions, comparable low levels of FcγR-dependent phagocytosis were observed on the Δ*TarM* and Δ*dltA* mutant cells (columns 16 and 20).

Also, when the number of phagocytosed bacteria by 100 PMNs was counted in the presence of anti-WTA IgG, the Δ*TarM* and Δ*dltA* mutant strains (avg. 535 ± 147 cells, columns 18 and 22) were engulfed by the PMNs, but not Δ*TarMS* (column 10), Δ*TarS* (column 14) and Δ*tagO* mutant strains (column 26). These results clearly indicate that anti-WTA antibody-mediated opsonophagocytosis was induced after the recognition of β-GlcNAc modification of WTA in *S. aureus*. This means β-GlcNAc modification of WTA is required for the induction of anti-WTA antibody-mediated classical complement, leading to opsonophagocytosis.

### Example 4: Affinity of Anti-WTA IgG for β-GlcNAc

To exclude the possibility of insufficient GlcNAc modification of the WTA in the *ΔTarS* mutant, the GlcNAc amount of the *ΔTarS* mutant strains obtained in Example 1 was measured.

### <4-1> Separation and Purification of WTA in S. aureus

The procedure of Example <2-1> was repeated using the strains obtained in Example 1 for separation and purification of WTA in *S. aureus*.

### <4-2> PAGE Analysis of WTA in Mutant Strain

Purified WTAs obtained in Example <4-1> (10 µg) were separated by PAGE (27%) and visualized by silver staining.

Specifically, PAGE was performed on a gel, and the gel was placed in a container using distilled water and then stained with 0.5% Alcian Blue in 5% acetic acid for 5 minutes. After removing Alcian Blue, the stained gel was washed for 40 minutes by immersing in distilled water. The gel was then fixed using a buffer (methanol 400 mL, acetic acid 100 mL and distilled water 500 mL) for 2 hours. The fixed gel was placed in 40 mL of 1X silver oxidizer (BioRad) and subjected to a reaction for 5 minutes, and then washed for 3 minutes 5 times. Then, the gel was placed in 40 mL of 1X silver reagent (BioRad) and subjected to a reaction for 20 minutes, followed by removal of the reagent. Subsequently, the gel was subjected to a reaction in 50 mL of silver stain developer (prepared by dissolving 1.6 g of silver stain developer in 50 mL of distilled water) to detect the WTA fraction.

The results are shown in Fig. 7. As shown in Fig. 7, the WTA obtained from *ΔtagO* mutants (lane 2) did not show any signal in silver stain, indicating that there is no WTA in the mutants. Meanwhile, the WTA obtained from *ΔTarS* and *ΔTarM* single mutants (lane 3 and 4) migrated faster than that of the parental strain (lane 1), and the WTA obtained from *ΔTarMS* double mutant (lane 5) migrated faster than those of the parental strain and *ΔTarS* and *ΔTarM* single mutants. It can be concluded that these migrations were due to the fact that *ΔTarS* and *ΔTarM* single mutants lost one type of GlcNAc residue and *ΔTarMS* double mutant lost two types of GlcNAc residues.

### <4-3> Amount of GlcNAc of WTA in Mutant Strains

Amounts of GlcNAc of WTA in mutant strains were measured as below.

Specifically, the WTA obtained in Example <4-1> was subjected to acid hydrolysis with 6N HCl at 100°C for 3 hours *in vacuo* to avoid the degradation of GlcNAc residue in the oxygen. Then, the WTA was evaluated for the inorganic phosphate content according to the method disclosed in De Gubareff T et al., J. Biol. Chem., 223:377-388, 1956. Also, the amount of GlcNAc was measured according to the method disclosed in Enghofer E et al., Carbohydrate research, 76:233-238, 1979, and the ratio of GlcNAc/inorganic phosphate (GlcNAc/Pi) was calculated. The results are shown in Fig. 2.

**[Table 2]**

| WTA | Pi (nmole/µg) | GlcNAc (nmole/µg) | GlcNAc/Pi ratio |
|---|---|---|---|
| Parental strains | 1.62 | 1.26 | 0.78 |
| *ΔTarM* | 3.14 | 1.13 | 0.36 |
| *ΔTarS* | 2.00 | 1.43 | 0.72 |
| *ΔTarMS* | 2.87 | 0.03 | 0.01 |

As shown in Table 2 above, *ΔTarMS* double mutants did not contain GlcNAc residues. Also, the ratio of GlcNAc/Pi of the parental strains and *ΔTarS* mutants was determined as 0.78 and 0.72, respectively, indicating that the *ΔTarS* mutant strains have amounts of GlcNAc residues comparable to those of the parental strains. However, the *ΔTarS* mutant strains of Example 3 did not show anti-WTA IgG-mediated opsonophagocytosis. On the other hand, although the ratio of GlcNAc/Pi of *ΔTarM* mutant was 0.36 which was much lower than that of the parental strains, opsonophagocytosis was induced at a level similar to that of the parental strains.

These results clearly indicate that the impaired anti-WTA IgG-mediated opsonophagocytosis of the *ΔTarS* mutant is not due to insufficient amounts of the α-GlcNAc or β-GlcNAc residues.

### <4-4> Relative Affinity of WTA of Mutant Strain for Anti-WTA IgG by ELISA

Relative affinity of WTAs obtained from *S. aureus* mutants for anti-WTA IgG was determined by ELISA.

Specifically, each WTA (5 nmol phosphate) in 60 µL of PBS (pH 7.5) was applied to F96 Cert. maxisorp immune plates (triplicate, Nunc) in triplicate, and the plates were incubated overnight at room temperature. The WTA-coated microplates were incubated with adequate volume of human serum in 50 µL of a buffer (10 mM Tris-HCl (pH 7.4), 140 mM NaCl, and 1% BSA) at 4°C for 2 hours, and bound IgGs were detected with mouse monoclonal anti-human-IgG antibodies (Sigma-Aldrich) and goat F(ab')₂ anti-mouse IgG (H+L) antibodies conjugated with horseradish peroxidase (HRP; Beckman Coulter, 1:10000 dilution). The plates were developed with the substrate 3,3',5,5'-tetramethylbenzidine (Zymed Laboratories) in dark conditions, and the development reaction was stopped by the addition of 2N H₂SO₄. Absorbance at 450 nm was recorded using a microplate reader (Thermo Scientific USA). Each anti-WTA IgG concentration was estimated compared to that of the control.

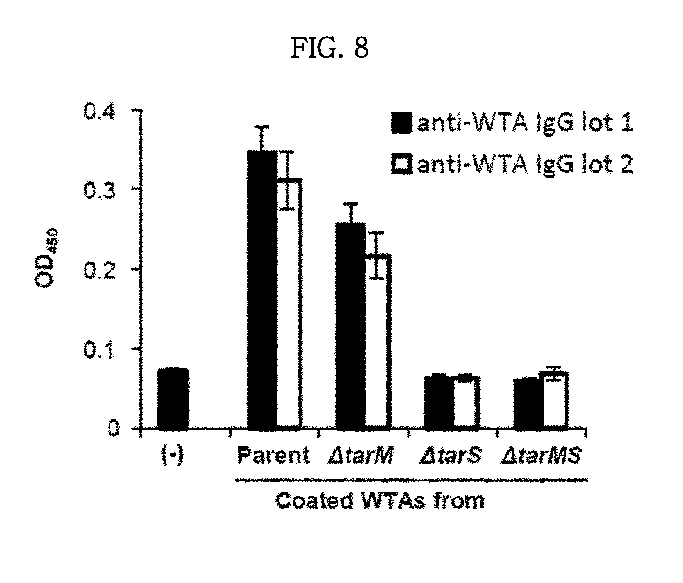

The results are shown in Fig. 8. As shown in Fig. 8, the β-GlcNAc-depleted WTAs from the *ΔTarS* mutants and *ΔTarMS* double mutants did not show binding to the purified serum anti-WTA IgGs. In contrast, the β-GlcNAc-sufficient WTAs from *ΔTarM* showed similar levels of binding ability as that of the parental strains. These results further confirm the binding specificity of serum anti-WTA antibodies to β-GlcNAc WTA.

### Example 5: Analysis of Anti-WTA Antibodies in Human Sera

Human sera and commercially available human intravenous IgGs (IVIG) were evaluated for the presence of anti-WTA antibodies specific for β-GlcNAc or α-GlcNAc modified WTA. Six adult sera were collected from healthy adults and two commercially available IVIGs (IV-Globulin-S, Green Cross Corporation, Korea; LIV-Gamma, SK chemical, Korea) were purchased.

Meanwhile, the amounts of anti-WTA IgG, anti-*ΔTarM* WTA IgG, anti-*ΔTarS* WTA IgG and anti-*ΔTarMS* WTA IgG were measured.

Specifically, the WTAs separated in Example <2-1> were dissolved in 1x PBS (5 nmol phosphate/50 µL concentration) and coated on a 96-well plate by introducing 50 µL of the solution thus prepared per well and stored at room temperature for 2 days. The TWA coated 96-well plate added with 200 µL of blocking buffer (20mM Tris 150 mM NaCl pH 7.4 and 1% BSA) and blocked at room temperature for 2 hours. The microplate was washed with 200 µL of washing buffer (10 mM Tris 140 mM NaCl pH 7.4) at room temperature for 1 minute. Subsequently, each of human serum to be tested was added with incubation buffer (10 mM Tris 140 mM NaCl pH 7.4 and 1% BSA) to make a total volume of 50 µL. The samples thus prepared were introduced into each well and allowed to react at 4°C for 2 hours. Upon completion of reaction, the microplate was washed with 200 µL of washing buffer for 30 seconds 5 times. The samples were subjected to a reaction at 4°C for 1 hour by adding 50 µL of monoclonal anti-human-IgG antibodies (Sigma-Aldrich, 1:1000 dilution) to each well, and then the microplate was washed with 200 µL of washing buffer for 30 seconds at room temperature 5 times. Then, the samples were subjected to a reaction at 4°C for 1 hour by adding 50 µL of goat F(ab')₂ anti-mouse IgG (H+L) antibodies conjugated with horseradish peroxidase (HRP) (Beckman Coulter, 1:10000 dilution) to each well, and then washed with washing buffer for 30 seconds at room temperature 5 times. After washing, the samples were subjected to a reaction for 10 minutes by adding 100 µL of tetramethyl benzidine (TMB) to each well, and the reaction was terminated by adding 100 µL of 2N sulfuric acid was added to each well1. Subsequently, absorbance at 450 nm was recorded using a microplate reader (Thermo scientific).

The results are shown in Table 3 below.

**[Table 3]**

| Serum No. | Sex | Age | Anti-WTA IgG (ng/µL) | Anti-*ΔTarM* WTA IgG | Anti*-ΔTarS* WTA IgG | Anti-*ΔTarMS* WTA IgG |
|---|---|---|---|---|---|---|
| Adult-1 | M | 28 | 610 | 400 | < 5 | < 5 |
| Adult-2 (A-2) | F | 26 | 460 | 450 | 24 | < 5 |
| Adult-3 (A-3) | F | 24 | 540 | 350 | 22 | < 5 |
| Adult-4 (A-4) | M | 28 | 420 | 340 | < 5 | < 5 |
| Adult-5 (A-5) | F | 26 | 540 | 480 | < 5 | < 5 |
| Adult-6 (A-6) | M | 30 | 700 | 510 | 19 | < 5 |
| Avg. | | | 550 (100%) | 420 (76%) | 22 | < 5 |
| IVIG-1 | NA | NA | 33 | 27 | 8.6 | <4 |
| IVIG-2 | NA | NA | 32 | 25 | 8.9 | <4 |
| Avg. | | | 33 (100%) | 26 (70%) | 8.8 | <4 |

As shown in Table 3 above, A-1 to A-6 sera contained anti-WTA IgG. When the amounts of anti β-GlcNAc WTA- or anti α-GlcNAc WTA-specific IgGs were estimated by ELISA using purified WTAs, an average of 76% of the total anti-WTA IgGs, which were specific to the WTA from the *S. aureus* parental strains, bound to the β-GlcNAcylated WTA. The average amounts of the serum IgGs specific to the α,β-GlcNAcylated WTA and β-GlcNAcylated WTA of six adults were calculated as 550 ng/µL and 420 ng/µL, respectively. An average of 14% of the IgG showed specific binding to the α-GlcNAcylated WTA and undetectable levels to the non-glycosylated WTA, which was purified from *ΔTarMS* double mutant strains. Additionally, two commercially available IVIGs contained 70% of the anti-WTA IgG specific to the β-GlcNAc WTA (average 26 ng/µL) among the anti-WTA-α,β-GlcNAc specific IgGs (33 ng/µL). These results strongly suggest that human sera contain β-GlcNAc WTA-specific antibodies as major *S. aureus* anti-WTA antibodies.

## Claims

1. A vaccine composition for preventing *staphylococcus aureus* infection comprising a native form of WTA containing a ribitol-phosphate which has been modified by *N*-acetylglucosamine (GlcNAc), which comprises, as an active ingredient:
(i) a ribitol-phosphate which has been modified only by a β-configuration in GlcNAc and not by α-configuration;
(ii) a repeating unit of the ribitol-phosphate which has been modified only by a β-configuration in GlaNAc and not by α-configuration; or
(iii) WTA containing the repeating unit of the ribitol-phosphate which has been modified only by a β-configuration in GlaNAc and not by α-configuration.

2. The vaccine composition for preventing *staphylococcus aureus* infection of claim 1, wherein (i) the ribitol-phosphate, (ii) the repeating unit of the ribitol-phosphate and (iii) the WTA containing the repeating unit are obtained by inhibiting *TarM*, thereby preventing the ribitol-phosphate of WTA from being modified by α-configuration in GlcNAc.

3. The vaccine composition for preventing *staphylococcus aureus* infection of claim 1 or 2, wherein the *staphylococcus aureus* infection is an infection caused by a kidney dialysis catheter or an infection caused by a surgical operation, a skin and soft tissue infection (SSTI), pneumonia, septicemia or acute respiratory tract infection.

4. The vaccine composition for preventing *staphylococcus aureus* infection of claim 1, wherein the *staphylococcus aureus* is methicillin-resistant *S. aureus* (MRSA) or pathogenic *S*. *aureus*.

5. A method for preparing anti-WTA antibodies of *S. aureus*, which comprises the steps of:
(1) preparing a mutant *S. aureus* containing WTA which has been modified only by a β-configuration in GlaNAc and not by α-configuration by mutating *S. aureus* with a native form of WTA containing GlcNAc-substituted ribitol-phosphate;
(2) separating (i) ribitol-phosphate which has been modified only by a β-configuration in GlaNAc and not by α-configuration; (ii) a repeating unit of the ribitol-phosphate which has been modified only by a β-configuration in GlaNAc and not by α-configuration; and (iii) WTA containing the repeating unit of the ribitol-phosphate which has been modified only by a β-configuration in GlaNAc and not by α-configuration from the mutant *S. aureus* and purifying them; and
(3) preparing anti-WTA antibodies by using the ribitol-phosphate, the repeating unit of the ribitol-phosphate or the WTA containing the repeating unit of the ribitol-phosphate as an antigen.

6. The method of claim 5, wherein Step (1) is accomplished by inhibiting glycosyltransferase *TarM* to prevent α-GlcNAc-modification of ribitol-phosphate in WTA.

7. The method of claim 6, wherein the inhibition of glycosyltransferase *TarM* is accomplished by deletion of *TarM* gene, which encodes glycosyltransferase *TarM.*

8. The method of claim 5, wherein the preparation of anti-WTA antibodies by using the ribitol-phosphate, the repeating unit of the ribitol-phosphate or the WTA containing the repeating unit of the ribitol-phosphate is immunization by inoculating the separated and purified ribitol-phosphate, the repeating unit of the ribitol-phosphate or the WTA containing the repeating unit of the ribitol-phosphate to a mammal.

9. A method for preventing *staphylococcus aureus* infection, which comprises administering to a mammal the separated and purified ribitol-phosphate, the repeating unit of the ribitol-phosphate or the WTA containing the repeating unit obtained in Step (2) or the anti-WTA antibodies obtained in Step (3).

10. The method of claim 9, wherein the *staphylococcus aureus* infection is an infection caused by a kidney dialysis catheter or an infection caused by a surgical operation, a skin and soft tissue infection (SSTI), pneumonia, septicemia or acute respiratory tract infection.

11. The method of claim 9, wherein the *staphylococcus aureus* is methicillin-resistant *S. aureus* (MRSA) or pathogenic *S. aureus*.

12. A method for preparing a vaccine composition for preventing *staphylococcus aureus* infection using the separated and purified ribitol-phosphate, the repeating unit of the ribitol-phosphate or the WTA containing the repeating unit of the ribitol-phosphate obtained in Step (2) or the anti-WTA antibodies obtained in Step (3).


SEQUENCE LISTINGS

<110>    MOGAM BIOTECHBOLOGY RESEARCH INSTITUTE
         PUSAN NATIONAL UNIVERSITY INDUSTRY-UNIVERSITY COOPERATION FOUNDATION

<120>    VACCINE COMPOSITION FOR PREVENTING STAPHYLOCOCCUS AUREUS
         INFECTION

<130>    PCB304015

<150>    US 61/643,697
<151>    2012-05-07

<160>    10

<170>    KopatentIn 2.0

<210>    1
<211>    30
<212>    DNA
<213>    Artificial Sequence

<220>
<223>    Primer spa-P1


<400>    1
gggtctagaa aaaagtcaag cctgaagtcg                                        30


<210>    2
<211>    30
<212>    DNA
<213>    Artificial Sequence

<220>
<223>    Primer spa-P2


<400>    2
tattggatcc aaagtggggc tttgaatgtg                                        30


<210>    3
<211>    30
<212>    DNA
<213>    Artificial Sequence

<220>
<223>    primer spa-P3


<400>    3
cccgggtacc tgcagcgtta ttagctggac                                        30

<210> 4
<211> 29
<212> DNA
<213> Artificial Sequence

<220>
<223> primer spa-P4

<400> 4
ggggaattct aattggtgca actgggaca 29

<210> 5
<211> 21
<212> DNA
<213> Artificial Sequence

<220>
<223> primer Phleo-P1

<400> 5
ggatccaata gaccagttgc a 21

<210> 6
<211> 21
<212> DNA
<213> Artificial Sequence

<220>
<223> primer Phleo-P3

<400> 6
ggtacccggg cgattgctga a 21

<210> 7
<211> 30
<212> DNA
<213> Artificial Sequence

<220>
<223> Primer SA0248-F-BamHI

<400> 7
caactggatc caaattctgg tggtccaggt 30

<210> 8
<211> 31
<212> DNA

<213>    Artificial Sequence

<220>
<223>    Primer SA0248-R-EcoRI


<400>    8
tttcgaattc gcgtagtgca acaatggtcg t                                    31


<210>    9
<211>    20
<212>    DNA
<213>    Artificial Sequence

<220>
<223>    Primer tarM-F1-HindIII


<400>    9
atgatgcgga catacctgct                                                 20


<210>    10
<211>    31
<212>    DNA
<213>    Artificial Sequence

<220>
<223>    Primer tarM-R2-BamHI


<400>    10
cacggatcct aaatgcaccc gtatcatcga a                                    31